# EUROPEAN PATENT APPLICATION

(11) **EP 4 082 539 A1**
(43) Date of publication of application: **02.11.2022**
(21) Application number: 20906479.9
(22) Date of filing: 25.12.2020
(51) Int. Cl.: A61K 31/19, A23L 2/52, A23L 33/105, A61P 25/28, A61P 43/00

(54) **NEW USES FOR STEVIOL**

(30) Priority: 27.12.2019 JP 2019238969
(71) Applicant: Suntory Holdings Limited, Osaka 530-8203 (JP)
(72) Inventor: YOSHIDA Junki, Kawasaki-shi, Kanagawa 211-0067 (JP); OHKURI Tadahiro, Kawasaki-shi, Kanagawa 211-0067 (JP); NAGAO Koji, Kawasaki-shi, Kanagawa 211-0067 (JP); YOKOO Yoshiaki, Kawasaki-shi, Kanagawa 211-0067 (JP)
(74) Representative: Mewburn Ellis LLP
(86) International application number: PCT/JP2020/049040
(87) International publication number: WO 2021/132698

(57) **Abstract**

The present invention provides compositions containing steviol and/or steviol glycoside, the compositions being for suppressing endoplasmic reticulum stress, promoting neurite growth, improving nerve damage, improving neurodegenerative disease, and/or promoting nerve regeneration.

## Description

### TECHNICAL FIELD

The present invention relates to novel use of steviol, particularly, use of steviol relating to suppression of endoplasmic reticulum stress, promotion of neurite elongation, improvement of nerve injury, improvement of neurodegenerative disease, and promotion of nerve regeneration, etc.

### BACKGROUND ART

While a wide variety of proteins are produced in cells in order to support life activity, most of membrane proteins and secretory proteins are folded and matured in the endoplasmic reticulum. However, if the amount of proteins sent into the endoplasmic reticulum exceeds the processing capacity of the endoplasmic reticulum for some reason, the folding of proteins in the endoplasmic reticulum becomes abnormal so that incompletely folded proteins accumulate in the endoplasmic reticulum, causing endoplasmic reticulum stress. Cells upon perceiving the occurrence of endoplasmic reticulum stress cause adaptive response called unfolded protein response (UPR) and normalize functions of the endoplasmic reticulum. However, too large endoplasmic reticulum stress causes apoptosis. The endoplasmic reticulum stress is involved in various diseases such as neurodegenerative disease, multiple sclerosis, glioma, diabetes mellitus, obesity, multiple myeloma, autoimmune disease, steatohepatitis, and insulin resistance (Non-Patent Literature 1). A method for treating a disease by targeting a molecule related to unfolded protein response is also under development (Non-Patent Literature 2).

Meanwhile, neuronal cells play an important role in controlling various functions of a living body. Matured neuronal cells have poor regeneration ability, making nerve tissue repair difficult. Neuronal cells are connected to each other through neurites to form a network. If the network is cut by neurite damage due to trauma or disease, major damage occurs in neural activity. In such a case, a substance that promotes elongation of neurites is considered useful in the improvement of damage, and search for such a substance is ongoing (Non-Patent Literature 3).

### CITATION LIST

### PATENT LITERATURE

Non-Patent Literature 1: Dufey et al., Am J Physiol Cell Physiol. 2014;307(7):C582-94
Non-Patent Literature 2: Almanza et al., FEBS J. 2019;286(2):241-278
Non-Patent Literature 3: More et al., Molecules. 2012;17(6):6728-53

### SUMMARY OF INVENTION

### TECHNICAL PROBLEM

The development of a further approach of suppressing endoplasmic reticulum stress, and a further approach of promoting neurite elongation are expected.

### MEANS FOR SOLVING THE PROBLEMS

In one embodiment, the present invention provides the following.

[1] A composition for use in the suppression of endoplasmic reticulum stress, the composition comprising steviol and/or steviol glycoside.

[1-1] A composition for use in the suppression of endoplasmic reticulum stress, the composition comprising steviol and/or steviol glycoside as active ingredient.

[2] The composition for use according to [1] or [1-1] for use in the treatment of a condition involving endoplasmic reticulum stress.

[3] A composition for use in the promotion of neurite elongation, the composition comprising steviol and/or steviol glycoside.

[3-1] A composition for use in the promotion of neurite elongation, the composition comprising steviol and/or steviol glycoside as active ingredient.

[4] The composition for use according to [3] or [3-1] for use in the treatment of a condition that is improved by promotion of neurite elongation

[5] A composition for use in the improvement of nerve injury, in the improvement of neurodegenerative disease and/or in the promotion of nerve regeneration, the composition comprising steviol and/or steviol glycoside.

[5-1] A composition for use in the improvement of nerve injury, in the improvement of neurodegenerative disease and/or in the promotion of nerve regeneration, the composition comprising steviol and/or steviol glycoside as active ingredient.

[6] The composition for use according to [5] or [5-1] for use in the suppression or improvement of organic change in neurodegenerative disease

[7] The composition for use according to any one of [1] to [6] for oral ingestion.

[8] The composition for use according to any one of [1] to [7] which is a beverage.

### ADVANTAGEOUS EFFECTS OF INVENTION

The present invention provides a further approach for suppressing endoplasmic reticulum stress, protecting cells and promoting neurite elongation, and increases options for preserving or enhancing health and preventing or improving a condition involving endoplasmic reticulum stress or neurite damage. Also, steviol can be orally administered as steviol glycoside which may be employed as a sweetener and has high compatibility with foods. Therefore, steviol can be ingested more conveniently as compared with conventional drugs, and can contribute to health enhancement for a wider range of people.

### BRIEF DESCRIPTION OF DRAWINGS

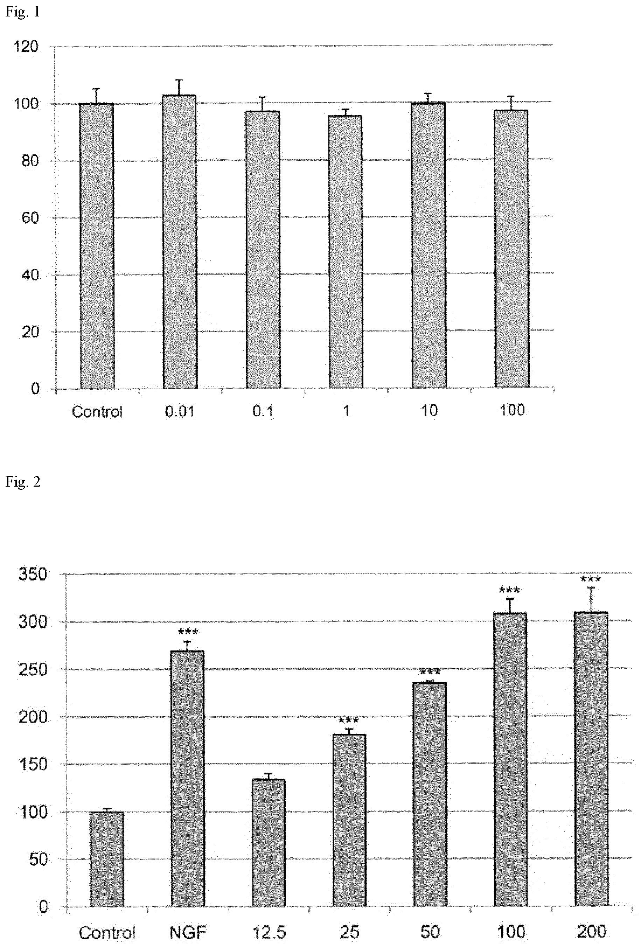

Figure 1 is a diagram showing results of a cytotoxicity test of steviol to PC12 cells (n = 3). The ordinate depicts absorbance difference (relative viable cell count (Control = 100%)), and the numerical values on the abscissa depict the concentrations (µg/mL) of steviol.
Figure 2 is a diagram showing a neurite elongation-promoting effect of steviol (n = 5). The ordinate depicts a relative neurite length when the value of negative control group (Control) is defined as 100%. The numerical values on the abscissa depict the concentrations (µg/mL) of steviol, and "NGF" depicts positive control group. ^{∗∗∗} represents P < 0.001 based on the Dunnett test.

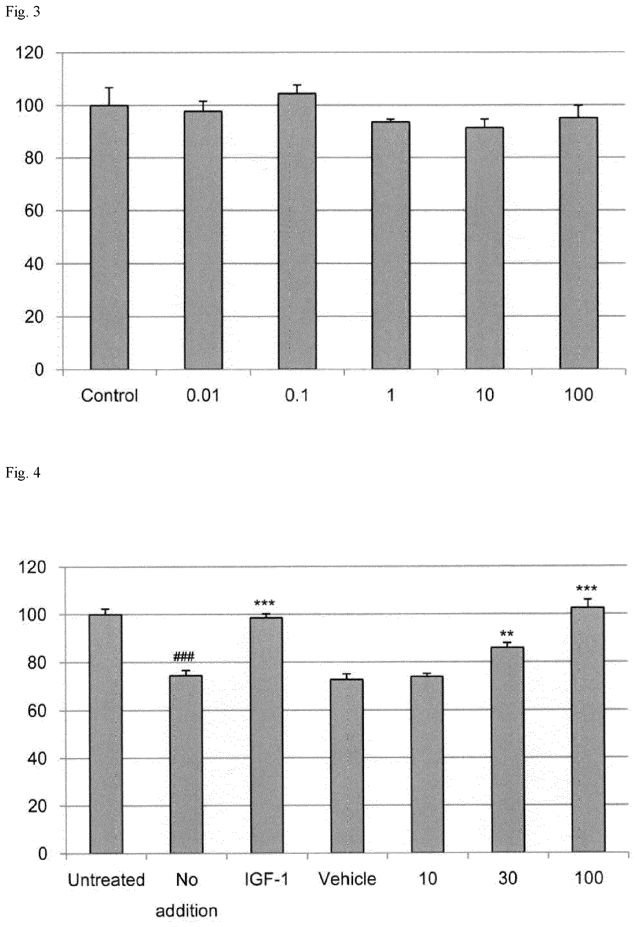

Figure 3 is a diagram showing results of a cytotoxicity test of steviol to neuronal cells (n = 3). The ordinate depicts absorbance difference (relative viable cell count), and the numerical values on the abscissa depict the concentrations (µg/mL) of steviol.
Figure 4 is a diagram showing a cell-protecting effect of steviol against endoplasmic reticulum stress (n = 5). The ordinate depicts a relative cell survival rate when the value of untreated control group (Untreated) is defined as 100%. The numerical values on the abscissa depict the concentrations (µg/mL) of steviol, "No addition" depicts no-addition control group, "IGF-1" depicts positive control group, and "Vehicle" depicts vehicle control group. ### and ^{∗∗∗} represent P < 0.001 based on the Student's t test, and ^{∗∗} represents P < 0.005 based on this test.

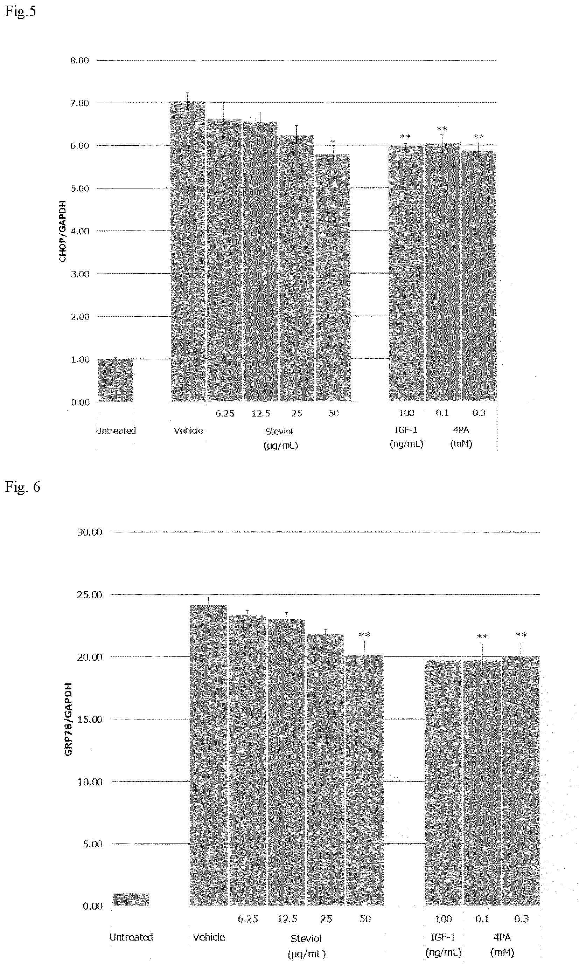

Figure 5 is a diagram showing steviol-mediated change in expression level of CHOP in neuronal cells induced to have endoplasmic reticulum stress. In the abscissa, "Untreated" depicts untreated control group, and "Vehicle "depicts vehicle control group. The ordinate depicts a relative expression level of CHOP to the expression level of GAPDH when the value of untreated control group is defined as 1. ^{∗}P < 0.05 vs. Vehicle, ^{∗∗}P < 0.01 vs. Vehicle, Dunnett test.
Figure 6 is a diagram showing steviol-mediated change in expression level of GRP78 in neuronal cells induced to have endoplasmic reticulum stress. In the abscissa, "Untreated" depicts untreated control group, and "Vehicle" depicts vehicle control group. The ordinate depicts a relative expression level of GRP78 to the expression level of GAPDH when the value of untreated control group is defined as 1. ^{∗∗}P < 0.01 vs. Vehicle, Dunnett test.

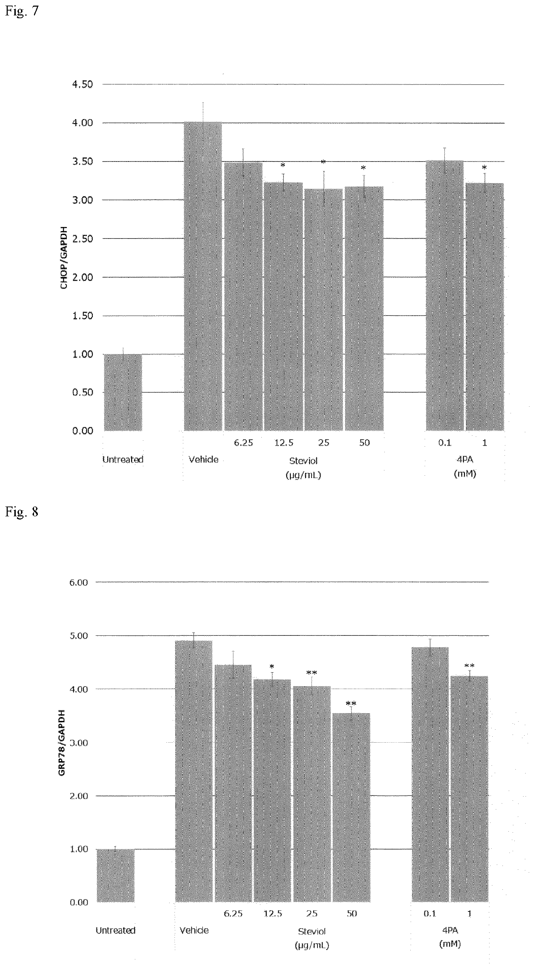

Figure 7 is a diagram showing steviol-mediated change in expression level of CHOP in adipocytes induced to have endoplasmic reticulum stress. In the abscissa, "Untreated" depicts untreated control group, and "Vehicle" depicts vehicle control group. The ordinate depicts a relative expression level of CHOP to the expression level of GAPDH when the value of untreated control group is defined as 1. ^{∗}P < 0.05 vs. Vehicle, Dunnett test.
Figure 8 is a diagram showing steviol-mediated change in expression level of GRP78 in adipocytes induced to have endoplasmic reticulum stress. In the abscissa, "Untreated" depicts untreated control group, and "Vehicle" depicts vehicle control group. The ordinate depicts a relative expression level of GRP78 to the expression level of GAPDH when the value of untreated control group is defined as 1. ^{∗}P < 0.05 vs. Vehicle, ^{∗∗}P < 0.01 vs. Vehicle, Dunnett test.

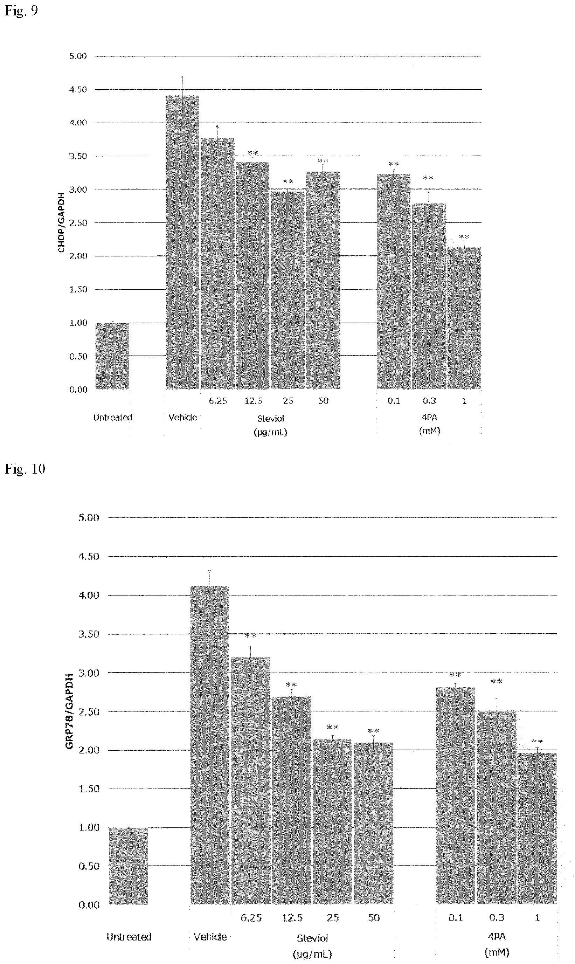

Figure 9 is a diagram showing steviol-mediated change in expression level of CHOP in hepatocytes induced to have endoplasmic reticulum stress. In the abscissa, "Untreated" depicts untreated control group, and "Vehicle "depicts vehicle control group. The ordinate depicts a relative expression level of CHOP to the expression level of GAPDH when the value of untreated control group is defined as 1. ^{∗}P < 0.05 vs. Vehicle, ^{∗∗}P < 0.01 vs. Vehicle, Dunnett test.
Figure 10 is a diagram showing steviol-mediated change in expression level of GRP78 in neuronal cells induced to have endoplasmic reticulum stress. In the abscissa, "Untreated" depicts untreated control group, and "Vehicle" depicts vehicle control group. The ordinate depicts a relative expression level of GRP78 to the expression level of GAPDH when the value of untreated control group is defined as 1. ^{∗∗}P < 0.01 vs. Vehicle, Dunnett test.

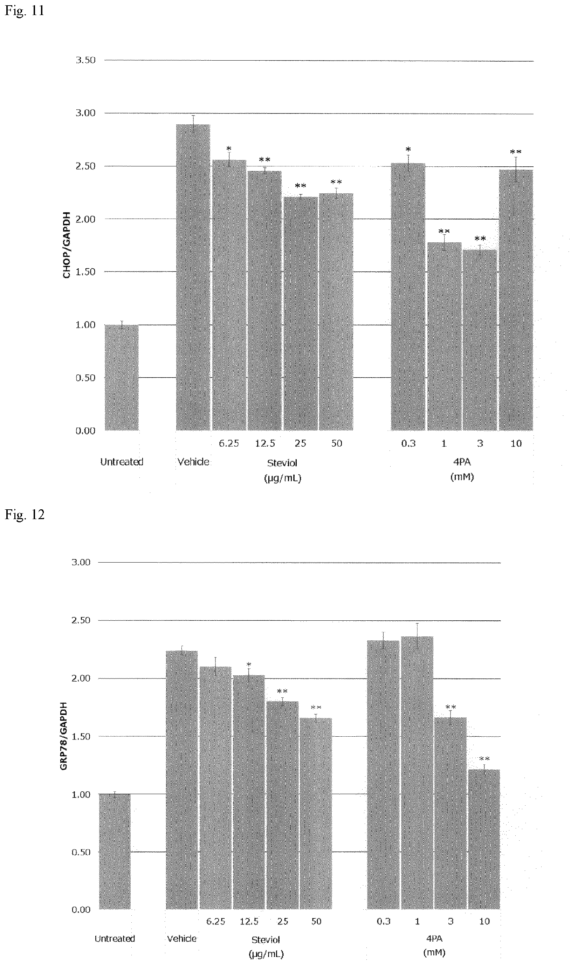

Figure 11 is a diagram showing steviol-mediated change in expression level of CHOP in pancreatic cells induced to have endoplasmic reticulum stress. In the abscissa, "Untreated" depicts untreated control group, and "Vehicle" depicts vehicle control group. The ordinate depicts a relative expression level of CHOP to the expression level of GAPDH when the value of untreated control group is defined as 1. ^{∗}P < 0.05 vs. Vehicle, ^{∗∗}P < 0.01 vs. Vehicle, Dunnett test.
Figure 12 is a diagram showing steviol-mediated change in expression level of GRP78 in pancreatic cells induced to have endoplasmic reticulum stress. In the abscissa, "Untreated" depicts untreated control group, and "Vehicle" depicts vehicle control group. The ordinate depicts a relative expression level of GRP78 to the expression level of GAPDH when the value of untreated control group is defined as 1. ^{∗}P < 0.05 vs. Vehicle, ^{∗∗}P < 0.01 vs. Vehicle, Dunnett test.

### DESCRIPTION OF EMBODIMENTS

The present invention will now be described in detail. The following embodiments are provided for illustrating the present invention and are not intended to limit the present invention only thereto. The present invention may be implemented in various forms, without departing from the spirit of the present invention.

Note that all documents, as well as laid-open application publications, patent application publications, and other patent documents cited herein shall be incorporated herein by reference. The present specification incorporates the contents of the specification and the drawings of Japanese Patent Application No. 2019-238969, filed on December 27, 2019, from which the present application claims priority.

### <Composition for suppression of endoplasmic reticulum stress>

One embodiment of the present invention relates to a composition for use in the suppression of endoplasmic reticulum stress, the composition comprising steviol and/or a source of steviol (e.g., steviol glycoside) (hereinafter, also referred to as the "composition for use in the suppression of endoplasmic reticulum stress of the present invention" or the "composition A of the present invention").

The present inventors have found for the first time that steviol has an endoplasmic reticulum stress-suppressing effect, and completed the composition A of the present invention. Thus, steviol and a source thereof can act as an active ingredient in the composition A of the present invention. The action as an active ingredient in the composition A of the present invention can be confirmed by, for example, more suppressed endoplasmic reticulum stress by the use of the composition A of the present invention than the use of a control composition when the composition A of the present invention is compared with the control composition which has the same composition as that of the composition A of the present invention except that the control composition is free of steviol and/or a source thereof.

Steviol is an aglycon of steviol glycoside contained in Stevia rebaudiana.

The source of steviol includes a substance capable of providing steviol by the action of an in vivo enzyme (which includes an enzyme produced by an in vivo bacterium or the like) when administered to a living body. Examples of the source of steviol include, but are not limited to, steviol glycoside and glucuronic acid conjugate of steviol (steviol glucuronide). The steviol glycoside is glycoside in which steviol is bonded to one or more sugars (e.g., glucose, rhamnose, and xylose). Non-limiting examples of steviol glycoside include, for example, rebaudioside A, rebaudioside B, rebaudioside D, rebaudioside E, rebaudioside F, rebaudioside I, rebaudioside J, rebaudioside K, rebaudioside M, rebaudioside N, rebaudioside O, rebaudioside Q, rebaudioside R, dulcoside A, rubusoside, steviolmonoside, steviolbioside and stevioside. In a preferred embodiment, the steviol glycoside includes a substance having good quality of taste, for example, rebaudioside D and rebaudioside M. In the present specification, rebaudioside is also abbreviated to "Reb", "Reb." or "R". In the present specification, for example, rebaudioside A is also referred to as "RebA", "Reb.A" or "RA". The steviol glycoside, when orally ingested, is degraded into steviol by large intestinal bacteria and absorbed into the body. Thus, the steviol glycoside serves as a source of steviol via the large intestine.

Commercially available steviol and source of steviol (e.g., steviol glycoside) may be employed. Steviol and a source of steviol extracted, purified, etc. from Stevia rebaudiana may be employed. An approach of extracting and purifying steviol and steviol glycoside from Stevia rebaudiana is known. Steviol and steviol glycoside can be extracted from a fresh leaf or a dried leaf of Stevia rebaudiana with a suitable solvent (an aqueous solvent such as water or an organic solvent such as alcohol, ether and acetone) (for extraction conditions, etc., see a method described in, for example, Ohta et al., J. Appl. Glycosci. 2010;57(3):199-209 or WO 2010/038911). Steviol and each type of steviol glycoside can be purified from the extracts thus obtained by use of a method known in the art such as gradient of ethyl acetate or any other organic solvent:water, high-performance liquid chromatography (HPLC), gas chromatography, time-of-flight mass spectrometry (TOF-MS), or ultra-performance liquid chromatography (UPLC). Steviol can be obtained by, for example, deglycosylation of steviol glycoside (e.g., Shibata et al., Plant Physiol. 1991;95(1):152-6). For steviol glycoside, particularly, with a small content in Stevia rebaudiana, it is possible to elevate its concentration by bioconversion which allows glycosyltransferase (e.g., UDP-sugar dependent glycosyltransferase (UGT)) to act on Stevia rebaudiana extracts containing steviol glycoside.

In one embodiment, the steviol glycoside to be employed in the composition A of the present invention is extracted and purified from Stevia rebaudiana without bioconversion. In another embodiment, the steviol glycoside to be employed in the composition A of the present invention is extracted and purified from Stevia rebaudiana by an approach including bioconversion.

It is more preferred that steviol and the source of steviol (e.g., steviol glycoside) to be employed in the composition A of the present invention should have a higher purity. Examples of the purity of steviol or the source of steviol (e.g., steviol glycoside) to be employed in the present invention include 90% or higher, 90.5% or higher, 91% or higher, 91.5% or higher, 92% or higher, 92.5% or higher, 93% or higher, 93.5% or higher, 94% or higher, 94.5% or higher, 95% or higher, 95.5% or higher, 96% or higher, 96.5% or higher, 97% or higher, 97.5% or higher, 98% or higher, 98.5% or higher, 99% or higher, and 99.5% or higher. For example, a peak area ratio in chromatography such as HPLC or GC may be used as an index for the purity of steviol or the source of steviol (e.g., steviol glycoside).

The composition A of the present invention may comprise steviol, a source of steviol (e.g., steviol glycoside), or a combination of steviol and a source of steviol (e.g., steviol glycoside). The blending ratio of steviol, the source of steviol (e.g., steviol glycoside), or the combination of steviol and the source of steviol (e.g., steviol glycoside) in the composition A of the present invention can be, for example, 1 to 500,000, 1 to 400,000, 1 to 300,000, 1 to 200,000, 1 to 100,000, 1 to 80,000, 1 to 60,000, 1 to 40,000, 1 to 20,000, 1 to 10,000, 1 to 8,000, 1 to 6,000, 1 to 4,000, 1 to 2,000, 1 to 1500, 1 to 1200, 5 to 1200, 1 to 1000, 5 to 1000, 10 to 1000, 1 to 900, 5 to 900, 10 to 900, 15 to 900, 20 to 900, 25 to 900, 30 to 900, 35 to 900, 40 to 900, 45 to 900, 50 to 900, 55 to 900, 1 to 800, 5 to 800, 10 to 800, 15 to 800, 20 to 800, 25 to 800, 30 to 800, 35 to 800, 40 to 800, 45 to 800, 50 to 800, 55 to 800, 1 to 700, 5 to 700, 10 to 700, 15 to 700, 20 to 700, 25 to 700, 30 to 700, 35 to 700, 40 to 700, 45 to 700, 50 to 700, 55 to 700, 1 to 600, 5 to 600, 10 to 600, 15 to 600, 20 to 600, 25 to 600, 30 to 600, 35 to 600, 40 to 600, 45 to 600, 50 to 600, 55 to 600, 1 to 500, 5 to 500, 10 to 500, 15 to 500, 20 to 500, 25 to 500, 30 to 500, 35 to 500, 40 to 500, 45 to 500, 50 to 500, 55 to 500, 1 to 400, 5 to 400, 10 to 400, 15 to 400, 20 to 400, 25 to 400, 30 to 400, 35 to 400, 40 to 400, 45 to 400, 50 to 400, 55 to 400, 1 to 300, 5 to 300, 10 to 300, 15 to 300, 20 to 300, 25 to 300, 30 to 300, 35 to 300, 40 to 300, 45 to 300, 50 to 300, 55 to 300, 1 to 250, 5 to 250, 10 to 250, 15 to 250, 20 to 250, 25 to 250, 30 to 250, 35 to 250, 40 to 250, 45 to 250, 50 to 250, 55 to 250, 1 to 200, 5 to 200, 10 to 200, 15 to 200, 20 to 200, 25 to 200, 30 to 200, 35 to 200, 40 to 200, 45 to 200, 50 to 200, 55 to 200, 1 to 180, 5 to 180, 10 to 180, 15 to 180, 20 to 180, 25 to 180, 30 to 180, 35 to 180, 40 to 180, 45 to 180, 50 to 180, 55 to 180, 1 to 550, 1 to 540, 1 to 530, 1 to 520, 1 to 510, 1 to 505, 1 to 495, 1 to 490, 5 to 550, 5 to 540, 5 to 530, 5 to 520, 5 to 510, 5 to 505, 5 to 500, 5 to 495, 5 to 490, 10 to 550, 10 to 540, 10 to 530, 10 to 520, 10 to 510, 10 to 505, 10 to 495, 10 to 490, 15 to 550, 15 to 540, 15 to 530, 15 to 520, 15 to 510, 15 to 505, 15 to 495 or 15 to 490 ppm based on the total mass of the composition A.

Endoplasmic reticulum stress means a state where the amount of proteins sent into the endoplasmic reticulum exceeds the processing capacity of the endoplasmic reticulum so that incompletely unfolded proteins accumulate in the endoplasmic reticulum. The suppression of endoplasmic reticulum stress usually includes suppression of occurrence of endoplasmic reticulum stress under conditions where endoplasmic reticulum stress occurs, and also includes suppression of an adverse effect (e.g., induction of apoptosis) of developed endoplasmic reticulum stress on cells, and protection of cells from endoplasmic reticulum stress (e.g., protection of cells from apoptosis associated with endoplasmic reticulum stress).

The suppression of endoplasmic reticulum stress by the composition A of the present invention can be evaluated by, for example, a reduced adverse effect of endoplasmic reticulum stress by the action of the composition A of the present invention on cells induced to have endoplasmic reticulum stress, as compared with no action of the composition A of the present invention. In a particular embodiment, the suppression of endoplasmic reticulum stress can be evaluated, as described in Examples mentioned later, by pretreating cells with the composition A of the present invention, then inducing endoplasmic reticulum stress with, for example, an endoplasmic reticulum stress inducer such as tunicamycin, and after a predetermined time (e.g., 24 hours later), quantifying the survival rate of the cells or the degree of apoptosis. The degree of suppression of endoplasmic reticulum stress may be, for example, 105% or more, 108% or more, 110% or more, 113% or more, 115% or more, 118% or more, 120% or more, 123% or more, 125% or more, 128% or more, 130% or more, 133% or more, 135% or more, 138% or more, 140% or more, 143% or more, 145% or more, 148% or more, or150% or more in terms of the survival rate of cells treated with the composition A of the present invention when the cell survival rate under endoplasmic reticulum stress induced under the same conditions as above except that the composition A of the present invention is not employed is defined as 100%. A higher value is more preferred.

The suppression of endoplasmic reticulum stress by the composition A of the present invention can also be evaluated by a reduced endoplasmic reticulum stress marker level by the action of the composition A of the present invention on cells induced to have endoplasmic reticulum stress, as compared with no action of the composition A of the present invention. In a particular embodiment, the suppression of endoplasmic reticulum stress can be evaluated, as described in Examples mentioned later, by pretreating cells with the composition A of the present invention, then inducing endoplasmic reticulum stress with, for example, an endoplasmic reticulum stress inducer such as tunicamycin, and after a predetermined time (e.g., 24 hours later), quantifying an endoplasmic reticulum stress marker in the cells. Examples of the endoplasmic reticulum stress marker include, but are not limited to, molecules related to endoplasmic reticulum stress, particularly, molecules related to apoptosis ascribable to endoplasmic reticulum stress, such as CHOP and GRP78. The degree of suppression of endoplasmic reticulum stress may be, for example, 97% or less, 95% or less, 94% or less, 93% or less, 91% or less, 90% or less, 89% or less, 88% or less, 86% or less, 85% or less, 84% or less, 83% or less, 81% or less, 80% or less, 79% or less, 78% or less, 76% or less, 75% or less, 74% or less, 73% or less, 71% or less, 70% or less, 69% or less, 68% or less, 66% or less, 65% or less, 64% or less, 63% or less, 61% or less, 60% or less, 59% or less, 58% or less, 56% or less, 55% or less, 54% or less, 53% or less, 51% or less, 50% or less, 49% or less, 48% or less, 46% or less, 45% or less, 44% or less, 43% or less, 41% or less, or 40% or less in terms of the amount of a marker detected in cells treated with the composition A of the present invention when the amount of the marker detected under endoplasmic reticulum stress induced under the same conditions as above except that the composition A of the present invention is not employed is defined as 100% as to a marker whose expression level is increased by endoplasmic reticulum stress. A lower value is more preferred.

The composition A of the present invention can be employed in treatment of various conditions involving endoplasmic reticulum stress. Thus, the present invention also relates to a composition for treating a condition involving endoplasmic reticulum stress, the composition comprising steviol and/or a source of steviol (e.g., steviol glycoside). In this context, the condition includes a disease, and a condition that is not far enough to be diagnosed as a disease, but is not healthy. Examples of the condition involving endoplasmic reticulum stress include, but are not limited to, neurodegenerative disease, multiple sclerosis, tumor such as glioma and multiple myeloma, diabetes mellitus, obesity, autoimmune disease, fibrous disease (e.g., pulmonary fibrosis, hepatic fibrosis, liver cirrhosis, chronic kidney disease, cardiac fibrosis, systemic or localized scleroderma, and ovarian fibrosis), steatohepatitis, insulin resistance, sarcopenia, inflammatory bowel disease, Crohn's disease, ulcerous colitis, osteogenesis imperfecta, polycystic ovarian syndrome, and viral infection (see, e.g., Dufey et al., Am J Physiol Cell Physiol. 2014;307(7):C582-94, Oakes & Papa, Annu Rev Pathol. 2015;10:173-94, Deldicque, Front Physiol. 2013;4:236, Malhi & Kaufman, J Hepatol. 2011;54(4):795-809, Kaser and Blumberg, Semin Immunol. 2009;21(3):156-63, Lisse et al., PLoS Genet. 2008;4(2):e7, Takahashi et al., Sci Rep. 2017;7(1):10824, Kropski & Blackwell, J Clin Invest. 2018;128(1):64-73, Yoshida, FEBS J. 2007;274(3):630-58, Fung and Liu, Annu Rev Microbiol. 2019;73:529-557, and Fung and Liu, Front Microbiol. 2014;5:296).

The neurodegenerative disease includes Alzheimer's disease, Lewy body dementia, Parkinson's disease, parkinsonian syndrome, amyotrophic lateral sclerosis (ALS), Huntington's disease, Huntington's disease-like syndrome, dentatorubropallidoluysian atrophy (DRPLA), spinal and bulbar muscular atrophy (SBMA), spinocerebellar ataxia, Machado-Joseph disease, Cerebellar ataxia cayman type, spastic ataxia of Charlevoix-Saguenay, autosomal recessive spinocerebellar ataxias, prion disease, ataxia telangiectasia, ataxia telangiectasia-like disorder, Alexander disease, Refsum disease, Friedreich's ataxia, Leber hereditary optic neuropathy, Cockayne syndrome, progressive supranuclear palsy, frontotemporal lobar degeneration, frontotemporal dementia and amyotrophic lateral sclerosis, Canavan disease, Rett syndrome, spinal muscular atrophy, McLeod syndrome, neurodegeneration with brain iron accumulation, aceruloplasminemia, Kufor-Rakeb syndrome, pantothenate kinase-associated neurodegeneration, HARP syndrome, Fazio-Londe syndrome, agenesis of the corpus callosum with peripheral neuropathy, band-like calcification with simplified gyration and polymicrogyria, Perry syndrome, familial amyloidosis, benign hereditary chorea, pontocerebellar hypoplasia, mitochondrial myopathy, sideroblastic anemia, primary lateral sclerosis, multiple neuropathy, aminoacylase 1 deficiency, early-onset spastic paralysis, infantile bilateral striatal necrosis, familial dementia, cerebral amyloid angiopathy, cerebellar ataxia, mental retardation, and dysequilibrium syndrome (CAMRQ), familial encephalopathy with neuroserpin inclusion bodies, giant axonal neuropathy, Marinesco-Shogren syndrome, neurodegeneration due to cerebral folate transport deficiency, chorea-acanthocytosis, Guillain-Barre syndrome, Fisher syndrome, subacute myelo-optico-neuropathy, multiple system atrophy, spinocerebellar degeneration, postherpetic neuralgia, subacute sclerosing panencephalitis, neuroferritinopathy, hereditary diffuse leukoencephalopathy with spheroid, PNPLA6-related disease, Brown-Vialetto-Van Laere syndrome (BVVLS), posterior column ataxia with retinitis pigmentosa, episodic ataxia, nuclear group neuroacanthocytosis, ataxia-oculomotor apraxia, distal hereditary motor neuropathy, leukoencephalopathy with vanishing white matter, progressive leukoencephalopathy with ovarian failure, leukoencephalopathy with brain stem and spinal cord involvement and brain lactate elevation, leukoencephalopathy with dystonia and motor neuropathy, megalencephalic leukoencephalopathy with subcortical cysts, cystic leukoencephalopathy without megalencephaly, coenzyme Q10 deficiency, global cerebral hypomyelination, autosomal-dominant striatal degeneration, familial Idiopathic basal ganglia calcification, Gordon Holmes syndrome, PEHO syndrome, PEHO-like syndrome, early-onset progressive encephalopathy with brain atrophy and thin corpus callosum (PEBAT), early-onset progressive encephalopathy with brain edema and/or leukoencephalopathy (PEBEL), nonprogressive cerebellar ataxia with mental retardation, spastic paraplegia-psychomotor retardation-seizures syndrome, and the like. In one embodiment, the neurodegenerative disease is accompanied by accumulation of abnormal proteins in neuronal cells.

Examples of the viral infection include, but are not limited to, infections with induction of endoplasmic reticulum stress, such as coronavirus infection (e.g., SARS, MERS, and COVID-19), hepatitis B, hepatitis C, hepatitis D, flavivirus infection, and Borna disease.

In one embodiment, the condition involving endoplasmic reticulum stress is a disease other than diabetes mellitus or its complications. In another embodiment, the condition involving endoplasmic reticulum stress is a condition caused by apoptosis ascribable to endoplasmic reticulum stress. It is a disease on which insulin therapy has low efficacy. In another embodiment, the composition A of the present invention is employed in a subject without diabetes mellitus that responds to insulin therapy (e.g., type 1 diabetes mellitus). In another embodiment, the composition A of the present invention is employed in a subject that manifests insulin resistance. In a particular embodiment, the composition A of the present invention is employed for suppressing or improving organic change (injury, loss, cell death (including apoptosis), etc., of neuronal cells) in neurodegenerative disease. The endoplasmic reticulum stress-suppressing effect of steviol revealed this time are not mediated by insulin. Therefore, the composition A of the present invention can exert its effects in an insulin-independent manner. Thus, the composition A of the present invention may be employed even for the disease, as described above, on which insulin cannot be expected to have a therapeutic effect.

### <Composition for promotion of neurite elongation>

Another embodiment of the present invention relates to a composition for use in the promotion of neurite elongation, the composition comprising steviol and/or a source of steviol (e.g., steviol glycoside) (hereinafter, also referred to as the "composition for use in the promotion of neurite elongation of the present invention" or the "composition B of the present invention").

The present inventors have found for the first time that steviol has a neurite elongation-promoting effect, and completed the composition B of the present invention. Thus, steviol and a source thereof can act as an active ingredient in the composition B of the present invention. The action as an active ingredient in the composition B of the present invention can be confirmed by, for example, more promoted neurite elongation by the use of the composition B of the present invention than the use of a control composition when the composition B of the present invention is compared with the control composition which has the same composition as that of the composition B of the present invention except that the control composition is free of steviol and/or a source thereof.

The origin, purity, the amount blended, etc., of steviol and the source of steviol (e.g., steviol glycoside) in the composition B of the present invention are as described above about the composition A of the present invention.

Neurite elongation means that a neurite (including a dendrite and/or an axon process) elongates in neuronal cells or neural progenitor cells (e.g., undifferentiated PC12 cells). The promotion of neurite elongation by the composition B of the present invention can be evaluated by change in index for neurite elongation as compared with the case where the composition B of the present invention is not employed. Examples of the index for neurite elongation include neurite length, surface area of neuronal cells, and numerical value related thereto (e.g., absorbance of a well containing stained neuronal cells). In a particular embodiment, the promotion of neurite elongation can be confirmed, as described in Examples mentioned later, by culturing neural progenitor cells for a predetermined time (e.g., several days, preferably 3 days) in a medium containing a differentiation inducer such as NGF in the presence of the composition B of the present invention to induce their differentiation into neuronal cells, then staining the cell membranes, and measuring absorbance. The rate of change in index (the rate of increase in absorbance in the method using cell membrane staining) may be, for example, 20% or more, 30% or more, 40% or more, 50% or more, 60% or more, 70% or more, 80% or more, 90% or more, 100% or more, 110% or more, 120% or more, 130% or more, 140% or more, 150% or more, 160% or more, 170% or more, 180% or more, 190% or more, 200% or more, 210% or more, 220% or more, 230% or more, 240% or more, or 250% or more based on an index obtained under the same conditions as above except that the composition B of the present invention is not employed. A larger value is more preferred.

The composition B of the present invention can be employed in treatment of a condition that is improved by promotion of neurite elongation. Thus, the present invention also relates to a composition for treating a condition that is improved by promotion of neurite elongation, the composition comprising steviol and/or a source of steviol (e.g., steviol glycoside). Examples of the condition that is improved by promotion of neurite elongation include, but are not limited to, nerve injury and neurodegenerative disease (see, e.g., More et al., Molecules. 2012;17(6):6728-53). The nerve injury includes central nerve injury and peripheral nerve injury. The neurodegenerative disease includes Alzheimer's disease, Lewy body dementia, Parkinson's disease, parkinsonian syndrome, amyotrophic lateral sclerosis (ALS), Huntington's disease, Huntington's disease-like syndrome, dentatorubropallidoluysian atrophy (DRPLA), spinal and bulbar muscular atrophy (SBMA), spinocerebellar ataxia, Machado-Joseph disease, Cerebellar ataxia cayman type, spastic ataxia of Charlevoix-Saguenay, autosomal recessive spinocerebellar ataxias, prion disease, ataxia telangiectasia, ataxia telangiectasia-like disorder, Alexander disease, Refsum disease, Friedreich's ataxia, Leber hereditary optic neuropathy, Cockayne syndrome, progressive supranuclear palsy, frontotemporal lobar degeneration, frontotemporal dementia and amyotrophic lateral sclerosis, Canavan disease, Rett syndrome, spinal muscular atrophy, McLeod syndrome, neurodegeneration with brain iron accumulation, aceruloplasminemia, Kufor-Rakeb syndrome, pantothenate kinase-associated neurodegeneration, HARP syndrome, Fazio-Londe syndrome, agenesis of the corpus callosum with peripheral neuropathy, band-like calcification with simplified gyration and polymicrogyria, Perry syndrome, familial amyloidosis, benign hereditary chorea, pontocerebellar hypoplasia, mitochondrial myopathy, sideroblastic anemia, primary lateral sclerosis, multiple neuropathy, aminoacylase 1 deficiency, early-onset spastic paralysis, infantile bilateral striatal necrosis, familial dementia, cerebral amyloid angiopathy, cerebellar ataxia, mental retardation, and dysequilibrium syndrome (CAMRQ), familial encephalopathy with neuroserpin inclusion bodies, giant axonal neuropathy, Marinesco-Shogren syndrome, neurodegeneration due to cerebral folate transport deficiency, chorea-acanthocytosis, Guillain-Barre syndrome, Fisher syndrome, subacute myelo-optico-neuropathy, multiple system atrophy, spinocerebellar degeneration, postherpetic neuralgia, subacute sclerosing panencephalitis, neuroferritinopathy, hereditary diffuse leukoencephalopathy with spheroid, PNPLA6-related disease, Brown-Vialetto-Van Laere syndrome (BVVLS), posterior column ataxia with retinitis pigmentosa, episodic ataxia, nuclear group neuroacanthocytosis, ataxia-oculomotor apraxia, distal hereditary motor neuropathy, leukoencephalopathy with vanishing white matter, progressive leukoencephalopathy with ovarian failure, leukoencephalopathy with brain stem and spinal cord involvement and brain lactate elevation, leukoencephalopathy with dystonia and motor neuropathy, megalencephalic leukoencephalopathy with subcortical cysts, cystic leukoencephalopathy without megalencephaly, coenzyme Q10 deficiency, global cerebral hypomyelination, autosomal-dominant striatal degeneration, familial Idiopathic basal ganglia calcification, Gordon Holmes syndrome, PEHO syndrome, PEHO-like syndrome, early-onset progressive encephalopathy with brain atrophy and thin corpus callosum (PEBAT), early-onset progressive encephalopathy with brain edema and/or leukoencephalopathy (PEBEL), nonprogressive cerebellar ataxia with mental retardation, spastic paraplegia-psychomotor retardation-seizures syndrome, and the like. In one embodiment, the neurodegenerative disease is accompanied by a neurite abnormality. The composition B of the present invention is employed for suppressing or improving organic change (injury, degeneration, loss, etc., of neurite) in neurodegenerative disease. The composition B of the present invention can also be used for promoting nerve regeneration.

The compositions A and B of the present invention (hereinafter, may be collectively referred to as the "composition of the present invention") may be a composition of any form, and may be, for example, a solid (e.g., powdery), gel-like, or liquid composition. Also, the composition of the present invention can be in various forms. The composition of the present invention can assume, for example, a form such as a food, a food additive, a medicament, a quasi-drug, or a cosmetic, or can be employed as a starting material for producing such a product. Thus, the present invention relates to a food, a food additive, a pharmaceutical product, a quasi-pharmaceutical product or a cosmetic to be employed in use selected from suppression of endoplasmic reticulum stress treatment of a condition involving endoplasmic reticulum stress, promotion of neurite elongation, treatment of a condition that is improved by promotion of neurite elongation and promotion of nerve regeneration, or a starting material of the above product, the product or the starting material comprising steviol and/or a source of steviol (e.g., steviol glycoside).

In the present invention, the food is a generic name for an eatable product, and includes a food prescribed in each country or region. The food according to the present invention includes, for example, a general food (which includes a health food such as a functional food, a dietary supplement, a health supplement, and an organic food), a food with health claims (a food for specified health use, a food with functional claims, a food with nutrient function claims, etc.), and a food for special dietary use (a food for specified health use, a food for sick people, dry milk for expectant and nursing mothers, an infant formula, a food for a dysphagic patient, etc.) as defined in Japan. The food according to the present invention also includes an infant food, a formula for a baby born prematurely, a food for aged people, a nursing care food, a medical food, etc.

Nutritional supplement foods refer to foods in which a specific nutritional ingredient is fortified. Health foods refer to foods that are healthful or are considered good for health, and include nutritional supplement foods, natural foods, diet foods. Functional foods refer to foods for supplying a nutritional ingredient that fulfills regulatory functions of the body. Foods for infants refer to foods that are provided to children aged up to about six. Geriatric foods refer to foods processed to be digested and absorbed more easily than non-processed foods. Infant milk formulas refer to milk formulas to be provided to infants aged up to about one. Premature infant milk formulas refer to milk formulas to be provided to premature infants until about six months after birth.

Forms of the foods are not particularly limited, and various forms may be taken. Examples of such forms include beverages, supplements, non-beverage foods, and the like. The beverages may be either of alcoholic beverages or non-alcoholic beverages.

Examples of the alcohol-free beverage include alcohol-free beer, malt beverages, lactic acid bacteria beverages, fermented beverages, cocoa beverages, sport drinks, energy drinks, tea-based beverages (e.g., tea beverages such as green tea, oolong tea, brown rice tea, and black tea, and herbal infusion beverages such as barley tea, Job's tears tea, sesame barley tea, buckwheat tea, cherry tea, Amacha (Japanese herbal tea made from fermented leaves of Hydrangea macrophylla var. thunbergii), and herbal tea), soft drinks, coffee beverages (packed coffee, liquid coffee, etc.), carbonated beverages (e.g., cola flavor beverages, clear carbonated beverages, ginger ale, fruit juice-based carbonated beverages, milk-containing carbonated beverages and sugar-free carbonated beverages), functional beverages (e.g., sport drinks, energy drinks, health support beverages and pouched jelly beverages), fruit juice/vegetable-based beverages (e.g., 100% fruit juice beverages, fruit-containing beverages, low-fruit juice content soft drinks, fruit grain-containing fruit beverages and fruit pulp beverages), dairy beverages (e.g., milk, drink yogurt, lactic acid bacteria beverages and milk-containing soft drinks), soy milk beverages (e.g., soy milk and soybean beverages), and flavor water.

Alcoholic beverages refer to beverages that contain alcohol raw material, and may be chuhai. Examples of the alcohol raw material include fermented liquors, distilled liquors, and mixed liquors. Examples of the fermented liquor include wine and beer. Examples of the distilled liquor include spirits (such as gin, vodka, rum, tequila, new spirits (alcoholic beverages mixed with less than 10% of unblended whiskey), and alcohol for raw material), liqueurs, whiskeys (such as whiskey and brandy), and shochu. Here, alcoholic beverages may be those containing alcohol at a detectable level and contain, for example, 1% by volume or more, 2% by volume or more, 3% by volume or more, 4% by volume or more, and 5% by volume or more of alcohol.

The beverage includes beverages having various calories. The beverage is preferably a low-calorie beverage (less than 20 kcal/100 mL) or a non-caloric beverage (less than 5 kcal/100 mL).

Non-beverage foods include, for example, a noodle (such as soba noodle, udon, Chinese noodle, instant noodle); tofu; a confectionery (such as a candy, a gum, a chocolate, a snack, a biscuit, a cookie, a gummy, a cake, a wafer, a sweet bun, a chocolate, and a Japanese sweet); a bread; a marine or livestock processed food (such as a kamaboko, a ham, and a sausage); a dairy product (such as a fermented milk, a butter, a cheese, a yogurt, and a ghee); a fat and oil, and an fat-and-oil processed food (such as a salad oil, a tempura oil, a margarine, and a mayonnaise, a shortening, a whipped cream, a dressing, and a fat spread); a condiment (such as a sauce and a baste); a cooked or semi-cooked product (such as a chanpuru); a retorted food (such as a curry, a stew, a bowl, a porridge, and a rice porridge); a chilled sweet (such as an ice cream, a sorbet, and a shaved ice); a powdered food (such as a powdered beverage and a powdered soup); and an enteric fluid food.

Preferable food in the present invention includes a beverage, a supplement, and the like.

The food additive is a product that is added to a food ingredient in producing a food, and is capable of assuming, for example, a form such as a powder, granules, a liquid or a paste.

Examples of the medicament include various dosage forms. Examples of the dosage form suitable for oral administration include, but are not limited to, powders, granules, tablets, capsules, solutions, suspensions, emulsions, gels, chewables, and syrups. Examples of the dosage form suitable for parenteral administration include injections (e.g., injectable solutions, injectable suspensions, injectable emulsions, and injections of type of preparation in use), ointments, creams, lotions, wet dressings, patches, eye drops, nasal drops, inhalants, and sprays.

Examples of the quasi-drug include, but are not limited to, forms such as dentifrices, mouth fresheners, diaphoretics, cosmeceuticals, hair coloring products, and sanitary napkins.

Examples of the cosmetic include, but are not limited to, face washes, makeup removers, cosmetic lotions, beauty essences, packs, and sunscreens.

The composition of the present invention may contain a component other than steviol and a source of steviol (e.g., steviol glycoside). Such a component may differ depending on the form, use, usage, etc., of the composition of the present invention. Non-limiting examples thereof include a sweetener, an excipient, a binder, a disintegrant, a coating agent, a lubricant, a colorant, a taste and flavor modifier, a stabilizer, an absorption enhancer, a pH adjusting agent, a preservative, an anti-oxidant, a fragrance, a vitamin, a trace metal ingredient, and the like.

Examples of the sweetener include natural sweeteners, sugar alcohols, artificial sweeteners, high-intensity sweeteners, and low-calorie sweeteners. More specific examples thereof include glucose, D-fructose, galactose, mannose, ribose, xylose, maltose, sucrose, lactose, arabinose, rare sugar (e.g., D-tagatose, D-sorbose, D-allulose (D-psicose), L-fructose, L-allulose (L-psicose), L-tagatose, L-sorbose, altrose, D-allose, talose, idose, and gulose), peptide-based sweeteners (such as aspartame, neotame and advantame); sucrose derivatives (such as sucralose); synthetic sweeteners (such as acesulfame K, saccharin, advantame, sodium cyclamate, dulcin), sugar alcohols (such as erythritol, xylitol, sorbitol, maltitol, and mannitol), monellin, curculin, brazzein, thaumatin; taumarin, mabinlin, brazzein, pentadin, hernandulcin, 4β-hydroxyhernandulcin, miraculin, glycyrrhizin, phyllodulcin; Siraitia grosvenorii (luo han guo) extract, Glycyrrhiza glabra (glycyrrhiza) extract, Rubus suavissimus S. Lee (tencha) extract, Hydrangea macrophylla var. thunbergii (sweet tea) extract, Sclerochiton ilicifolius extract, Thaumataococcus daniellii Benth (Katemfe) extract, Dioscoreophyllum volkensii (serendipity berry) extract, Curculigo latifolia (Curculigo) extract, Richadella dulcifica (miracle fruit) extract, Pentadiplandra brazzeana (white liana) extract, Capparis masaikai (Mabinlang) extract, Lippia dulcis (Mexican sweet herb) extract, neohesperidin dihydrochalcone, Palatinit, high fructose liquor; fructose-glucose liquor; glucose-fructose liquor; oligosaccharide; honey; sugar cane juice (brown sugar syrup); sugar (white sugar, soft brown sugar, brown sugar, refined Japanese sugar, and the like); maple syrup; molasses; and starch syrup.

In the case of employing the composition of the present invention as a food, a food additive may be contained as the further component. Examples of the food additive include an excipient (such as wheat starch, corn starch, cellulose, lactose, sucrose, mannitol, sorbitol, xylitol, a pregelatinized starch, casein, magnesium aluminate silicate, and calcium silicate); a binder (such as pregelatinized starch, hydroxypropyl methyl cellulose, and polyvinylpyrrolidone; a disintegrant such as cellulose, hydroxypropyl methyl cellulose, and corn starch); a fluidizer (such as light anhydrous silicic acid); a fat and oil (for example, a vegetable oil such as soybean oil, sesame oil, olive oil, linseed oil, perilla oil, rapeseed oil, coconut oil, and corn oil, or an oil derived from animals or fish); a nutrient (such as various kind of minerals, various kind of vitamins, and an amino acid); a flavor; a sweetener; a taste modifier; a colorant; a solvent (such as ethanol); salts; a pH adjusting agent; a buffer; an anti-oxidant; a stabilizer, a gelling agent; a thickener; a lubricant; an encapsulant; a suspending agent; a coating agent; a preservative; and an emulsifier. The food additive may be used singly or in combination of two or more.

In the case of employing the composition of the present invention as a medicament or a quasi-drug, pharmaceutically acceptable additives (e.g., surfactants, carriers, diluents, and excipients) may be contained as other components. The pharmaceutically acceptable additives are well known in the medical field, and described in, for example, Remington's Pharmaceutical Sciences, 18th Ed., Mack Publishing Co., Easton, PA (1990), which is incorporated herein by reference in its entirety.

The composition of the present invention can be produced, for example, by mixing steviol and/or a source of steviol (e.g., steviol glycoside), if necessary, with other components as mentioned above, and processing the resultant into a desired form.

The composition of the present invention can be employed in a manner suitable for each form. For example, the food can be orally ingested as well as can be ingested through a parenteral nutritional pathway such as a transnasal or enteral pathway. The food additive can be employed in a state contained in a food, through the same pathway as in the food. The medicament can be administered through an administration route appropriate for a dosage form, for example, a route such as, but not limited to, oral, buccal, intraoral, intravenous, intramuscular, subcutaneous, intracutaneous, local, rectal, intra-articular, intracapsular, intra-arterial, intraportal, intraventricular, transmucosal, percutaneous, intranasal, intraperitoneal, intra-airway, intratumoral, intracerebral, intrathecal, intracerebroventricular, intrapulmonary and intrauterine routes. Both the quasi-drug and the cosmetic can be employed by use methods appropriate for their forms, for example, application to the skin or gargling.

The intake amount (dose or dosage) of steviol and/or the source of steviol (e.g., steviol glycoside) is not particularly limited and can be appropriately selected according to the age, body weight, health condition, etc. of a recipient subject. The daily intake may be, for example, 0.001 mg or more (e.g., 0.001 to 10,000 mg), 0.01 mg or more (e.g., 0.05 to 5,000 mg), 0.1mg or more (e.g., 0.5 to 1,000 mg) or 1 mg or more (e.g., 5 to 500 mg).

The timing of use of the compositions of the present invention is not particularly limited, and may be, for example, before, during, after, or between meals, or before bedtime.

The product comprising the composition of the present invention may indicate each type of use or function. Examples of such an indication include suppression of endoplasmic reticulum stress, treatment of a condition involving endoplasmic reticulum stress, promotion of neurite elongation, treatment of a condition that is improved by promotion of neurite elongation and promotion of nerve regeneration, and indications that can be regarded as being equivalent thereto. The condition involving endoplasmic reticulum stress and the condition that is improved by promotion of neurite elongation are as described above with respect to the composition of the present invention.

Herein, the term "subject" means any individual organism, preferably animal, more preferably mammalian, further preferably human individual to which the composition of the present invention is able to provide a desired effect. The subject may be healthy (e.g., have no particular or any disease) or may be affected by some disease. When the treatment of a disease is intended, for example, the subject typically means a subject affected by the disease or having a risk of being affected by the disease.

Herein, the term "treatment" includes every type of medically acceptable prophylactic and/or therapeutic intervention aimed at cure, transient remission or prevention, etc. of a disease. The "treatment" includes medically acceptable intervention for various purposes including, for example, the delay or arrest of progression of a disease, the regression or disappearance of a lesion, the prevention of onset of the disease or the prevention of recurrence of the disease. Thus, the composition of the present invention can be used in the treatment and/or the prevention of a disease.

Another embodiment of the present invention relates to a method of suppressing endoplasmic reticulum stress, treating a condition involving endoplasmic reticulum stress, promoting neurite elongation, treating a condition that is improved by promotion of neurite elongation, or promoting nerve regeneration in a subject in need thereof, comprising administering an effective amount of steviol and/or a source of steviol (e.g., steviol glycoside) to the subject.

The effective amount of steviol and/or the source of steviol (e.g., steviol glycoside) in the method of the present invention includes an amount of steviol and/or the source of steviol (e.g., steviol glycoside) capable of achieving an effect related to the method, and can be determined by the evaluation approach described above with respect to the composition of the present invention, an experiment using a known animal model related to the target disease, or condition. In a particular embodiment, the effective amount of steviol and/or the source of steviol (e.g., steviol glycoside) in the method of the present invention can be, for example, 0.001 mg or more (e.g., 0.001 to 10,000 mg), 0.01 mg or more (e.g., 0.05 to 5,000 mg), 0.1mg or more (e.g., 0.5 to 1,000 mg) or 1 mg or more (e.g., 5 to 500 mg).

Other features, for example, steviol and the source of steviol (e.g., steviol glycoside), and the target disease or condition, in the method of the present invention are as described above with respect to the composition of the present invention.

Another embodiment of the present invention relates to steviol and/or a source of steviol (e.g., steviol glycoside) for use in the suppression of endoplasmic reticulum stress, in the treatment of a condition involving endoplasmic reticulum stress, in the promotion of neurite elongation, in the treatment of a condition that is improved by promotion of neurite elongation or in the promotion of nerve regeneration. Each feature according to this embodiment is as described above with respect to the composition of the present invention.

Another embodiment of the present invention relates to use of steviol and/or a source of steviol (e.g., steviol glycoside) in the manufacture of a medicament for suppressing endoplasmic reticulum stress, treating a condition involving endoplasmic reticulum stress, promoting neurite elongation, treating a condition that is improved by promotion of neurite elongation or promoting nerve regeneration. Each feature according to this embodiment is as described above with respect to the composition of the present invention.

### EXAMPLES

Hereinafter, Examples, etc. related to the present invention will be described. However, the present invention is not limited by these specific embodiments.

### [Example 1] Evaluation of cytotoxicity of steviol to PC12 cells

Steviol was obtained by the following approach. Sodium periodate was added to a container containing stevioside (Cat. No. S0594, Tokyo Chemical Industry or Cat. No. S623, AK Scientific) and purified water, and the mixture was stirred overnight at room temperature. Potassium hydroxide was added thereto at internal temperature of 5 to 10°C under ice cooling, and the mixture was heated at internal temperature of 100 to 102°C for 2 hours. After confirmation of the completion of the reaction by HPLC, acetic acid was added thereto at internal temperature of 10 to 13°C under ice cooling. Next, extraction with ethyl acetate was performed three times, and the obtained organic layer was washed with a 10% aqueous sodium sulfite solution and subsequently with saline. The organic layer was dried over anhydrous magnesium sulfate and then concentrated under reduced pressure at 40°C to obtain a crude product of a yellow solid. Methanol was added thereto, and the mixture was stirred for approximately 15 minutes. Then, purified water was added thereto, and a solid was collected by filtration. The solid collected by filtration was dried overnight at 50°C to obtain a white solid. This solid was dissolved in acetone at 50°C, followed by clarifying filtration. The filtrate was concentrated, and the obtained solid was dried under reduced pressure overnight at 50°C to obtain steviol of interest (purity: 98% (HPLC), acetone: 0.5 wt%). Steviol thus obtained was employed in the following experiments.

PC12 cells (clone HS, Cat. No. JCRB0266, JCRB) were precultured for 3 days in a collagen IV-coated flask (T-75, Corning Inc., Cat. No. 354523) using a growth medium (DMEM medium (low glucose, Cat. No. 08456-65, Nacalai Tesque, Inc.) supplemented with 10% FBS (Cat. No. 172012, Sigma-Aldrich Co., LLC), 10% horse serum (Cat. No. 16050-122, Thermo Fisher Scientific Inc.), and 1% penicillin-streptomycin) in a CO₂ incubator (37°C, 5% CO₂; the same holds true for the description below). The cells were detached using 0.25% trypsin-EDTA (Cat. No. 32777-44, Nacalai Tesque, Inc.), seeded at 2,000 cells/100 µL/well to a 96-well black plate (Cat. No. 655090, Greiner Bio-One International GmbH) coated with laminin (Cat. No. L2020, Sigma-Aldrich Co., LLC) using the growth medium, and cultured overnight in a CO₂ incubator (37°C, 5% CO₂; the same holds true for the description below). On the next day, the medium was replaced with 100 µL of a test medium (DMEM (low glucose) supplemented with 1 ng/mL NGF (Cat. No. 13257019, Thermo Fisher Scientific Inc.), 0.1% FBS, 0.1% horse serum, and 1% penicillin-streptomycin) supplemented with varying concentrations of steviol (0.01, 0.1, 1, 10 or 100 µg/mL, dissolved in 0.1% DMSO (final concentration)), and the cells were cultured for 3 days in a CO₂ incubator. A vehicle control composed of a test medium supplemented with 0.1% DMSO (final concentration) was used as a negative control. Viable cell count measurement was performed by the WST-8 method. Specifically, the medium of each well after culture was replaced with 100 µL of a differentiation medium (DMEM (low glucose) supplemented with 50 ng/mL NGF, 0.1% FBS, 0.1% horse serum, and 1% penicillin-streptomycin) supplemented with a 1/10 amount of viable cell count measurement reagent SF (Cat. No. 25300, Nacalai Tesque, Inc.), and the cells were incubated in a CO₂ incubator. 30 minutes later and 90 minutes later, absorbance (450 nm, reference wavelength: 630 nm) was measured using a plate reader (VARIOSKAN FLASH, Thermo Fisher Scientific Inc.), and absorbance difference per 60 minutes was regarded as a relative viable cell count (Control =100%). As shown in the results of Table 1 and Figure 1, no particular cytotoxicity to be noted was found when 100 µg/mL steviol was added.

**Table 1 Results of cytotoxicity to PC12 cells**

| | Control | Steviol (µg/mL) | | | | |
|---|---|---|---|---|---|---|
| | | 0.01 | 0.1 | 1 | 10 | 100 |
| Relative viable cell count (%) | 100 | 102.8 | 97.0 | 95.3 | 99.6 | 96.9 |
| Standard error | 5.2 | 5.4 | 5.1 | 2.3 | 3.5 | 5.1 |

### [Example 2] Evaluation of neurite elongation-promoting effect of steviol on PC12 cell

PC12 cells (clone HS) were precultured using a growth medium in the same manner as in Example 1. The cells were detached using 0.25% trypsin-EDTA, seeded at 2,000 cells/100 µL/well to a 96-well black plate coated with laminin using the growth medium, and cultured overnight in a CO₂ incubator. On the next day, the medium was replaced with a differentiation medium (100 µL) supplemented with varying concentrations of steviol (10, 30 or 100 µg/mL, dissolved in 0.1% DMSO (final concentration)), and the cells were cultured for 3 days in a CO₂ incubator. The cells thus cultured were subjected to cell membrane staining using Cell Membrane Stain attached to Neurite Outgrowth Staining Kit (Thermo Fisher Scientific Inc.) and nuclear staining using Hoechst 33342 (Cat. No. H3570). Specifically, the medium was replaced with 100 µL of a fixative and staining solution (prepared by adding a 1/1000 amount each of Cell Membrane Stain (1000×) and Hoechst 33342 and a 1/25 amount of a formaldehyde solution (Cat. No. 061-00416, FUJIFILM Wako Pure Chemical Corp.) to DPBS (++) (Cat. No. 14040133, Thermo Fisher Scientific Inc.)), followed by incubation for 20 minutes in a CO₂ incubator (5% CO₂, 37°C, humid). Next, the medium was replaced with 100 µL of a 1× background suppression dye (prepared by adding a 1/100 amount of Background Suppression Dye (100×) attached to Neurite Outgrowth Staining Kit to DPBS (++)). Photographing and quantitative analysis were carried out using Operetta CLS (Perkin Elmer, Inc.), and a neurite length per cell was calculated. Nine fields of view at the center of each well were photographed using a 10× objective lens. A test medium supplemented with 50 ng/mL NGF was used as a positive control, and a vehicle control composed of a differentiation medium supplemented with 0.1%DMSO (final concentration) was used as a negative control. Significant difference among the test groups to be compared (negative control group vs positive control group/steviol addition group) was tested by the Dunnett test. The two-tailed test was conducted, and P < 0.05 was regarded as having significant difference. As shown in the results of Table 2 and Figure 2, steviol promoted neurite elongation in a concentration-dependent manner.

**Table 2 Promotion of neurite elongation in PC12 cells**

| | Control | NGF | Steviol ( µg/mL) | | | | |
|---|---|---|---|---|---|---|---|
| | | | 12.5 | 25 | 50 | 100 | 200 |
| Relative neurite length (%) | 100 | 269.4 | 133.3 | 180.9 | 235.2 | 307.7 | 308.6 |
| Standard error | 3.5 | 9.8 | 6.3 | 6.0 | 2.1 | 15.4 | 25.9 |

### [Example 3] Evaluation of cytotoxicity of steviol to neuronal cells

PC12 cells (Cat. No. 88022401, ECACC) were precultured in a collagen IV-coated flask using the growth medium. The cells were detached using 0.25% trypsin-EDTA, seeded at 5,000 cells/100 µL/well to a collagen IV-coated 96-well plate (Cat. No. 354429, Corning) using the growth medium, and cultured overnight in a CO₂ incubator. On the next day, the medium was replaced with 100 µL of the differentiation medium, and the cells were cultured for 2 days in a CO₂ incubator to induce their differentiation into neuronal cells. Then, the medium was replaced with 100 µL of a differentiation medium supplemented with varying concentrations of steviol (0.01, 0.1, 1, 10 or 100 µg/mL, dissolved in 0.1% DMSO (final concentration)), and the cells were cultured for 2 days in a CO₂ incubator. A vehicle control composed of a test medium supplemented with 0.1% DMSO (final concentration) was used as a negative control. Viable cell count measurement was performed by the WST-8 method in a same manner as Example 1. As shown in the results of Table 3 and Figure 3, no particular cytotoxicity to be noted was found when 100 µg/mL steviol was added.

**Table 3 Results of cytotoxicity to PC12 cells**

| | Control | Steviol (µg/mL) | | | | |
|---|---|---|---|---|---|---|
| | | 0.01 | 0.1 | 1 | 10 | 100 |
| Relative viable cell count (%) | 100 | 97.7 | 104.4 | 93.6 | 91.4 | 95.1 |
| Standard error | 6.7 | 3.8 | 3.2 | 1.0 | 3.2 | 4.7 |

### [Example 4] Evaluation of cell-protecting effect of steviol against endoplasmic reticulum stress

PC12 cells were precultured using the growth medium in a same manner as Example 3. The cells were detached using 0.25% trypsin-EDTA, seeded at 5,000 cells/100 µL/well to a collagen IV-coated 96-well plate (Cat. No. 354429, Corning) using the growth medium, and cultured overnight in a CO₂ incubator. On the next day, the medium was replaced with 100 µL of the differentiation medium, and the cells were cultured for 2 days in a CO₂ incubator to induce their differentiation into neuronal cells. Next, the medium was replaced with 100 µL of a differentiation medium supplemented with varying concentrations of steviol (10, 30 or 100 µg/mL, dissolved in 0.1% DMSO (final concentration)), and the cells were pretreated by culture for 24 hours in a CO₂ incubator. Then, 100 µL of a differentiation medium containing a 2x concentration of tunicamycin (final concentration: 1 µM, Cat. No. T7765, Sigma-Aldrich Co., LLC) and the same concentrations as above of steviol was added to the cells, which were then cultured for 24 hours in a CO₂ incubator for endoplasmic reticulum stress induction treatment. The control groups used are shown in the table given below. IGF-1 (Cat. No. SRP4121, Sigma-Aldrich Co., LLC) was dissolved at 100 µg/mL in Nuclease Free Water and then added at 100 ng/mL to the differentiation medium.

**Table 4 Treatment of each control group**

| | Pretreatment | Endoplasmic reticulum stress induction treatment |
|---|---|---|
| Untreated control group | Differentiation medium | Differentiation medium |
| No addition control group | Differentiation medium | Differentiation medium + 1 µM tunicamycin |
| Vehicle control group | Differentiation medium + 0.1% DMSO | Differentiation medium + 0.1% DMSO + 1 µM tunicamycin |
| Positive control group | Differentiation medium + 100 ng/mL IGF-1 | Differentiation medium + 100 ng/mL IGF-1 + 1 µM tunicamycin |

After the completion of endoplasmic reticulum stress induction treatment, viable cell count measurement was performed by the WST-8 method to evaluate damage on the cells by endoplasmic reticulum stress. Specifically, the medium of each well after culture was replaced with 200 µL of a differentiation medium supplemented with a 1/10 amount of viable cell count measurement reagent SF, and the cells were incubated in a CO₂ incubator. 30 minutes later and 90 minutes later, absorbance (450 nm, reference wavelength: 630 nm) was measured using a plate reader (VARIOSKAN FLASH, Thermo Fisher Scientific Inc.), and absorbance difference per 60 minutes was regarded as a relative viable cell count. Significant difference among the test groups to be compared (untreated control group vs no-addition control group, no-addition control group vs positive control group, vehicle control group vs steviol treatment group) was tested (two-tailed) by the Student's t test, and P < 0.05 was regarded as having significant difference. As shown in the results of Table 5 and Figure 4, steviol suppressed an adverse effect of endoplasmic reticulum stress on cells in a concentration-dependent manner.

**Table 5 Cell protection by steviol against endoplasmic reticulum stress**

| | Untreated | No addition | IGF-1 | Vehicle | Steviol (µg/mL) | | |
|---|---|---|---|---|---|---|---|
| | | | | | 10 | 30 | 100 |
| Relative viable cell count (%) | 100 | 74.4 | 98.5 | 72.7 | 73.8 | 85.9 | 102.5 |
| Standard error | 2.3 | 2.1 | 1.6 | 2.3 | 1.3 | 2.0 | 3.4 |

### [Example 5] Evaluation of endoplasmic reticulum stress-suppressing effect of steviol

Steviol was evaluated for its influence on endoplasmic reticulum stress in each type of cell by using endoplasmic reticulum stress markers CHOP and GRP78 as an index. The test cells used were neuronal cells (induced from PC12 cells), adipocytes (normal human subcutaneous preadipocytes, Cat. No. PT-5020, Lonza Group AG), hepatocytes (HepG2, Cat. No. JCRB1054, JCRB) and pancreatic cells (βTC6, ATCC^{®} CRL-11506).

Neuronal cells were prepared as follows. PC12 cells were reconstituted by seeding at 2 × 10⁶ cells/flask to a collagen IV-coated flask, and precultured for 3 days in the growth medium in a CO₂ incubator. The cells were detached using 0.25% trypsin-EDTA, seeded at 10,000 cells/100 µL/well to a collagen IV-coated 96-well plate (Cat. No. 354429, Corning) using the growth medium, and cultured in a CO₂ incubator. On the next day, the medium was replaced with the differentiation medium, and the cells were cultured for 2 days in a CO₂ incubator to induce their differentiation into neuronal cells.

Adipocytes were prepared as follows. Normal human subcutaneous preadipocytes were reconstituted in a T75 flask using a growth medium (PBM-2 medium, Cat. No. PT-8002, Lonza, supplemented with 10% FBS, 2 mM L-glutamine, 50 µg/mL gentamycin, and 37 ng/mL amphotericin-B), and precultured for 3 days in a CO₂ incubator (37°C, 5% CO₂; the same holds true for the description below). The cells were detached using 0.25% trypsin-EDTA, seeded at 10,000 cells/100 µL/well to a 96-well plate using the growth medium, and cultured in a CO₂ incubator. On the next day, the medium was replaced with a differentiation medium (100 µL), and the cells were cultured for 7 days in a CO₂ incubator to induce their differentiation into adipocytes.

Hepatocytes were prepared as follow. HepG2 cells were seeded to a T75 flask and precultured for 1 day in a CO₂ incubator using a growth medium (DMEM (high glucose, Cat. No. D5796, Sigma-Aldrich) supplemented with 10% FBS (Cat. No. 172012, Sigma-Aldrich), 1% penicillin-streptomycin (Cat. No. 09367-34, Nacalai Tesque, Inc.)). The cells were detached using 0.25% trypsin-EDTA, seeded at 10,000 cells/100 µL/well to a 96-well plate using the growth medium, and cultured in a CO₂ incubator.

Pancreatic cells were prepared as follow. βTC6 cells were precultured for 1 day in a CO₂ incubator using a growth medium (DMEM (high glucose, Cat. No. D5796, Sigma-Aldrich) supplemented with 15% FBS (Cat. No. 172012, Sigma-Aldrich), 1% penicillin-streptomycin (Cat. No. 09367-34, Nacalai Tesque, Inc.)). The cells were detached using 0.25% trypsin-EDTA, seeded at **10,000** cells/100 µL/well to a 96-well plate using the growth medium, and cultured in a CO₂ incubator.

The medium of the cells of each line prepared as described above was replaced with 100 µL of a differentiation medium (for the neuronal cells and the adipocytes) or a growth medium (for the hepatocytes and pancreatic cells) supplemented with varying concentrations of steviol (6.25, 12.5, 25 or 50 µg/mL, dissolved in 0.1% DMSO (final concentration)), and the cells were pretreated by culture for the time shown in Table 6 in a CO₂ incubator. Then, 100 µL of a differentiation medium (for the neuronal cells and the adipocytes) or a growth medium (for the hepatocytes and pancreatic cells) containing the concentration shown in Table 6 of tunicamycin and the same concentrations as above of steviol was added to the cells, which were then cultured for 24 hours in a CO₂ incubator for endoplasmic reticulum stress induction treatment. The control groups used are shown in Table 7. IGF-1 was dissolved at 100 µg/mL in Nuclease Free Water and then added at 100 ng/mL to a differentiation medium (for only the neuronal cells). 4PA (P21005, Sigma-Aldrich Co., LLC) was dissolved in a medium and then added at a predetermined concentration to a differentiation medium or a growth medium.

**Table 6 Duration of pretreatment and concentration of tunicamycin**

| | Pretreatment (h) | | Tunicamycin (µM) | |
|---|---|---|---|---|
| | CHOP measurement group | GRP78 measurement group | CHOP measurement group | GRP78 measurement group |
| Neuronal cell | 1 | 24 | 0.16 | 1.0 |
| Adipocyte | 24 | 24 | 0.65 | 0.65 |
| Hepatocyte | 4 | 4 | 0.65 | 0.65 |
| Pancreatic cell | 4 | 4 | 0.16 | 0.16 |

**Table 7 Treatment of each control group**

| | Pretreatment | Endoplasmic reticulum stress induction treatment |
|---|---|---|
| Untreated control group | Differentiation medium or growth medium | Differentiation medium or growth medium |
| Vehicle control group | Differentiation medium or growth medium + 0.1% DMSO | Differentiation medium or growth medium + 0.1% DMSO + tunicamycin |
| Positive control group | Differentiation medium or growth medium + IGF-1 or 4PA | Differentiation medium or growth medium + IGF-1 or 4PA + tunicamycin |

After the completion of endoplasmic reticulum stress induction treatment, the expression levels of CHOP and GRP78 were measured to evaluate the degree of endoplasmic reticulum stress. First, FasteLane lysate (RNA) was prepared for RNA extraction using FastLane Cell Probe kit (Cat. No. 216413, Qiagen N.V.). After removal of the medium, each well was washed by the addition of 125 µL of Buffer FCW and its immediate removal. Then, 50 µL of Cell processing mix was added to each well, followed by incubation at room temperature for 5 minutes to prepare FasteLane lysate. Next, the FasteLane lysate was transferred to a PCR tube and incubated at 75°C for 5 minutes. The lysate was preserved at -80°C until use.

Gene expression analysis was carried out by the following procedures. One Step real-time RT-PCR was performed with the composition of Table 8 using QuantiTect Probe RT-PCR Kit attached to FastLane Cell Probe kit.

**Table 8 Composition of mixture for RT-PCR**

| Component | Volume (µL) |
|---|---|
| 2x QuantiTect Probe RT-PCR Master Mix | 10 |
| 20x TaqMan Gene Expression Assay | 1 |
| QuantiTect RT Mix | 0.2 |
| FastLane lysate | 2 |
| RNase-free water | 6.8 |
| Total | 20.0 |

TaqMan Gene Expression Assay of the following ID was used.

**Table 9 TaqMan Gene Expression Assay ID**

| Cell | Gene | ID |
|---|---|---|
| Neuronal cell | Rat CHOP | Rn00492098_g1 |
| | Rat GRP78 | Rn00565250_m1 |
| | Rat GAPDH | Rn01775763_g1 |
| Hepatocyte and adipocyte | Human CHOP | Hs00358796_g1 |
| | Human GRP78 | Hs00607129_gH |
| | Human GAPDH | Hs02786624_g1 |
| Pancreatic cell | Mouse CHOP | Mm01135937_g1 |
| | Mouse GRP78 | Mm00517691_m1 |
| | Mouse GAPDH | Mm99999915_g1 |

RT-PCR reaction was performed under conditions of 50°C for 30 minutes, 95°C for 15 minutes, and (94°C for 15 seconds and 60°C for 60 seconds) x 40 cycles. The internal standard gene used was GAPDH gene. A relative expression level was calculated by the ΔΔCt method. Significant difference among tunicamycin addition groups (vehicle group vs positive control group/steviol addition group) was tested by the Dunnett test. The two-tailed test was conducted, and P < 0.05 was regarded as having significant difference.

As shown in the results of Tables 10 to 13 and Figures 5 to 12, steviol suppressed endoplasmic reticulum stress in each type of cell in a concentration-dependent manner.

**Table 10 Expression level of CHOP and GRP78 in neuronal cells**

| | Untreated | IGF-1 | 4PA (mM) | | **Vehicle** | **Steviol (µg/mL)** | | | |
|---|---|---|---|---|---|---|---|---|---|
| | | | 0.1 | 0.3 | | 6.25 | 12.5 | 25 | 50 |
| CHOP | 1 | 5.97 | 6.04 | 5.87 | 7.04 | 6.61 | 6.55 | 6.24 | 5.79 |
| GRP78 | 1 | 19.77 | 19.72 | 20.05 | 24.15 | 23.31 | 23.00 | 21.84 | 20.15 |

**Table 11 Expression level of CHOP and GRP78 in adipocytes**

| | Untreated | 4PA (mM) | | Vehicle | **Steviol (µg/mL)** | | | |
|---|---|---|---|---|---|---|---|---|
| | | 0.1 | 1 | | 6.25 | 12.5 | 25 | 50 |
| CHOP | 1.00 | 3.52 | 3.22 | 4.01 | 3.49 | 3.23 | 3.15 | 3.17 |
| GRP78 | 1.00 | 4.78 | 4.25 | 4.91 | 4.46 | 4.18 | 4.06 | 3.54 |

**Table 12 Expression level of CHOP and GRP78 in hepatocytes**

| | Untreated | 4PA (mM) | | | Vehicle | **Steviol (µg/mL)** | | | |
|---|---|---|---|---|---|---|---|---|---|
| | | 0.1 | 0.3 | 1 | | 6.25 | 12.5 | 25 | 50 |
| CHOP | 1.00 | 3.23 | 2.79 | 2.13 | 4.41 | 3.76 | 3.40 | 2.97 | 3.27 |
| GRP78 | 1.00 | 2.82 | 2.49 | 1.96 | 4.11 | 3.19 | 2.70 | 2.14 | 2.10 |

**Table 13 Expression level of CHOP and GRP78 in pancreatic cells**

| | Untreated | 4PA (mM) | | | | Vehicle | **Steviol (µg/mL)** | | | |
|---|---|---|---|---|---|---|---|---|---|---|
| | | 0.3 | 1 | 3 | 10 | | 6.25 | 12.5 | 25 | 50 |
| CHOP | 1.00 | 2.53 | 1.78 | 1.71 | 2.47 | 2.89 | 2.56 | 2.46 | 2.21 | 2.25 |
| GRP78 | 1.00 | 2.33 | 2.37 | 1.66 | 1.22 | 2.24 | 2.10 | 2.03 | 1.80 | 1.66 |

Those skilled in the art will understand that many various modifications can be made in the present invention without departing from the spirit of the present invention. Thus, it should be understood that the modes of the present invention described in the present specification are given merely for illustrative purposes and are not intended to limit the scope of the present invention.

## Claims

1. A composition for use in the suppression of endoplasmic reticulum stress, the composition comprising steviol and/or steviol glycoside.

2. The composition for use according to claim 1 for use in the treatment of a condition involving endoplasmic reticulum stress.

3. A composition for use in the promotion of neurite elongation, the composition comprising steviol and/or steviol glycoside.

4. The composition for use according to claim 3 for use in the treatment of a condition that is improved by promotion of neurite elongation

5. A composition for use in the improvement of nerve injury, in the improvement of neurodegenerative disease and/or in the promotion of nerve regeneration, the composition comprising steviol and/or steviol glycoside.

6. The composition for use according to claim 5 for use in the suppression or improvement of an organic change in neurodegenerative disease

7. The composition for use according to any one of claims 1 to 6 for oral ingestion.

8. The composition for use according to any one of claims 1 to 7 which is a beverage.
